(19) Europäisches Patentamt
European Patent Office
Office européen des brevets

(11) Veröffentlichungsnummer: **0 306 707 B1**

# (12) EUROPÄISCHE PATENTSCHRIFT

(45) Veröffentlichungstag der Patentschrift: **27.12.90**

(51) Int. Cl.[5]: **C07D 307/08**

(21) Anmeldenummer: **88112702.1**

(22) Anmeldetag: **04.08.88**

## (54) Verfahren zur destillativen Reinigung von Tetrahydrofuran.

(30) Priorität: **12.08.87 DE 3726805**

(43) Veröffentlichungstag der Anmeldung: **15.03.89 Patentblatt 89/11**

(45) Bekanntmachung des Hinweises auf die Patenterteilung: **27.12.90 Patentblatt 90/52**

(84) Benannte Vertragsstaaten: **BE DE ES FR GB IT NL**

(56) Entgegenhaltungen:
**EP-A- 0 022 469**
**DE-A- 2 704 505**
**FR-A- 2 340 314**

**CHEMICAL ABSTRACTS, Band 102, Nr. 4, 28.01,1985, Columbus,Ohio, USA ABU-EISHAH et al. " Design and control of a two-column azeotropic distillation system ", Seite 141, Spalte 1, Z'fassung 27 045e.**

(73) Patentinhaber: **BASF Aktiengesellschaft, Carl-Bosch-Strasse 38, D-6700 Ludwigshafen(DE)**

(72) Erfinder: **Palm, Christof, Dr., Pariser Strasse 33, D-6700 Ludwigshafen(DE)**
Erfinder: **Huchler, Otto Hermann, Dr., Weinbietstrasse 38, D-6703 Limburgerhof(DE)**
Erfinder: **Pessel, Ulrich-Dieter, Muemmelmannweg 8, D-6900 Heidelberg(DE)**
Erfinder: **Hollborn, Erdmann, Jakobsgarten 8, D-6700 Ludwigshafen(DE)**
Erfinder: **Schmitt, Dieter, Dr., Suedtiroler Ring 36, D-6719 Weisenheim(DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein kontinuierliches Verfahren zur destillativen Reinigung von Tetrahydrofuran (THF).

Die Herstellung von THF durch Dehydratisierung von Butandiol-1.4 an sauren Katalysatoren wird z.B. in der DE-AS 25 09 968 und der DE-PS 2 930 144 beschrieben. Bei der Reaktion, die nach dem Formelschema:

$$C_4H_8(OH)_2 \rightarrow C_4H_8O + H_2O$$

verläuft, wird das Butandiol-1.4 in je ein Mol THF und Wasser gespalten.

Das bei der Reaktion gebildete THF und das in äquimolarer Menge mitentstandene Wasser, das 20 Gew.% des Gemisches ausmacht, werden üblicherweise aus dem Reaktionsgemisch abdestilliert und zur Ausschleusung des Wassers in einer Kolonne in das wasserärmere THF-Azeotrop und das Abwasser fraktioniert. Dann wird das THF-Azeotrop nach verschiedenen Verfahren weiter entwässert und das THF rein destilliert.

Nach einem in der US-PS 4 093 633 beschriebenen Verfahren wird die Gewinnung von reinem THF aus dem rohen wasserhaltigen THF durch eine destillative Aufarbeitung in drei hintereinander geschalteten Destillationskolonnen vorgenommen. Dabei wird das Wasser aus dem Sumpf der ersten Kolonne, die 5 bis 30 theoretische Böden aufweist und mit einem Rückflußverhältnis von 0,5 zu 5 betrieben wird, abgezogen. Das noch Wasser enthaltende Destillat, das man vom Kopf der ersten Kolonne seitlich in die zweite Kolonne einleitet, wird in der zweiten Kolonne, die 5 bis 30 theoretische Böden aufweist und mit einem Druck von 3 bis 20 $kg/cm^3$ und einem Rückflußverhältnis von 0,5 bis 5 betrieben wird, destillativ so aufgetrennt, daß man wasserhaltiges THF vom Kolonnenkopf in die erste Kolonne zurückführt und das im wesentlichen wasserfreie Sumpfprodukt in der dritten Kolonne rein destilliert. Die dritte Kolonne wird mit 10 bis 30 theoretischen Böden und mit einem Rückflußverhältnis von 0,5 bis 2 betrieben.

Das so erhaltene wasserfreie THF genügt in seiner Reinheit vielfältigen Verwendungszwecken. Es hat sich jedoch gezeigt, daß das mit dem THF hergestellte Polytetrahydrofuran (PTHF) die gestiegenen hohen Anforderungen, die man an die Farbzahl von PTHF stellt, nicht erfüllt.

Es war deshalb nach einem Verfahren zu suchen, das es gestattet, aus dem bei der THF-Synthese anfallenden wasserhaltigen Rohprodukt ein THF mit der geforderten hohen Reinheit zu gewinnen. Wie wir festgestellt haben, sind für die ungenügende Reinheit des nach den bekannten Methoden destillierten THF noch im THF verbliebene niedriger siedende Verunreinigungen verantwortlich zu machen, die sicher aus dem THF entfernt werden müssen. Das läßt sich durch eine weitere Destillation nur mit unbefriedigendem Ergebnis erreichen. Man benötigt außerdem zusätzlich sehr hohe Bodenzahlen bzw. Rücklaufverhältnisse und verliert viel THF mit der Überkopfausschleusung der Leichtsiederverunreinigungen in der Reinkolonne.

Es wurde nun gefunden, daß man bei der destillativen Reinigung von rohem wasserhaltigen THF, bei der man das rohe Tetrahydrofuran durch drei Destillationskolonnen leitet, wobei man Wasser aus dem Sumpf der ersten Kolonne abzieht, wasserhaltiges Tetrahydrofuran vom Kopf der zweiten Kolonne in die erste Kolonne zurückführt und aus dem Sumpfprodukt der zweiten Kolonne in der dritten Kolonne reines Tetrahydrofuran abdestilliert, ein THF mit der geforderten hohen Reinheit erhält, wenn man einen Seitenabzug der ersten Kolonne in die zweite Kolonne leitet, das Kopfprodukt der dritten Kolonne in die erste Kolonne zurückführt, am Kopf der ersten Kolonne ein Destillat entnimmt, und das reine Tetrahydrofuran aus dem Seitenabzug der dritten Kolonne gewinnt.

Das erfindungsgemäße Verfahren ermöglicht die Gewinnung eines hochreinen THF, wobei man für die erzielte sichere Entfernung von Leichtsiedern im Vergleich zur nächstbekannten unzureichenden Methode eine geringere Bodenzahl und kein erhöhtes Rücklaufverhältnis benötigt.

Nach dem neuen Verfahren nimmt man die destillative Reinigung des rohen wasserhaltigen THF in drei hintereinandergeschalteten Destillationskolonnen so vor, wie es z.B. aus der Figur ersichtlich ist. Dabei werden die Kolonnen auf an sich übliche Weise betrieben, und zwar die erste Kolonne (1) mit 30 bis 50 theoretischen Böden unter Atmosphärendruck und einem Rückflußverhältnis, bezogen auf den Seitenabzug (6), von 1,5 bis 2, die zweite Kolonne (2) mit 10 bis 30 theoretischen Böden, einem Druck von 4 bis 20 bar und einem Rückflußverhältnis von 1 bis 2 und die dritte Kolonne (3) mit 40 bis 50 theoretischen Böden, unter Atmosphärendruck und einem Rückflußverhältnis, bezogen auf den Seitenabzug, von 4 zu 8.

Das rohe wasserhaltige THF hat einen Wassergehalt von 18 bis 28 Gwe.% und enthält z.B. bis zu 5 Gew.% Verunreinigungen, die durch die bei der THF-Synthese verwendeten Rohstoffe bedingt sind, wie 2,3-Dihydrofuran und 2- und 3-Methyltetrahydrofuran. Es wird seitlich in die erste Kolonne eingeleitet. Die Zuleitung (4) befindet sich zweckmäßigerweise in der unteren Hälfte oberhalb des Sumpfes der Kolonne. Vom Sumpf der Kolonne wird Wasser ausgeschleust (5). Die Kolonne (1) enthält einen oberhalb der Zuleitung (4) angebrachten Seitenabzug (6), durch den man ein flüssiges THF/Wasser-Gemisch entnimmt und unter Druckerhöhung seitlich in die Druckkolonne (2) einleitet. Vom Kopf dieser Druckkolonne wird wasserhaltiges THF in die erste Kolonne zurückgeführt (7). Das Sumpfprodukt der zweiten Kolonne, das im wesentlichen wasserfrei ist, wird seitlich in die dritte Kolonne (8) eingeleitet. Schwersiedende Verunreinigungen werden dem Sumpf der dritten Kolonne entnommen (9).

Erfindungsgemäß wird auch das Kopfprodukt der dritten Kolonne, das noch Reste von Wasser und Leichtsiedern enthält (10), in die erste Kolonne zurückgeführt, während man das hochreine THF dem seitenabzug der dritten Kolonne (11) entnimmt. Erfindungswesentlich ist auch, daß man am Kopf der ersten Kolonne ein Destillat entnimmt (12), das offensichtlich die für die Qualitätsminderung des reinen THF verantwortlichen Nebenprodukte enthält, welche bei der geläufigen destillativen Aufarbeitung von THF im Verfahrensprodukt verbleiben sind. Nach dem Verfahren dieser Erfindung betreibt man die erste Kolonne bevorzugt so, daß man am Kolonnenkopf ein Destillat rektifiziert, in dem der THF-Gehalt auf 50 bis 10 Gew.%, vorzugsweise 30 bis 10 Gew.%, abgereichert ist. Dieses aus dem Kreislauf entfernte Destillat wird z.B. verbrannt.

Die in dem Beispiel genannten Prozente sind Gewichtsprozente.

Beispiel

Es wurde die durch die Figur dargestellte Destillationsapparatur verwendet. Sie besteht aus den drei Kolonnen (1), (2) und (3), von denen die Kolonne (1) 40 und die Kolonne (3) 46 theoretische Böden aufweisen und bei Normaldruck betrieben werden. Kolonne (2), die bei erhöhtem Druck von 5 bar betrieben wird, hat 30 theoretische Böden. In die Kolonne (1) wurde über die Zuleitung (4) ein dampfförmiges Gemisch aus 80 Gew.% THF und 20 Gew.% Wasser, das als charakteristische leichtsiedende Verunreinigung 0,1% 2,3-Dihydrofuran und als charakteristische höhersiedende Verunreinigungen 0,5% 2- und 3-Methyltetrahydrofuran enthielt, eingeleitet. Gleichzeitig wurde der Kolonne (1) über Leitung (7) der dampfförmige Kopfabzug der Kolonne (2) und über Leitung (10) der flüssige Kopfabzug der Kolonne (3) zugeführt. Die Kolonne (1) wurde mit einem Seitenabzug (6), einem Kopfabzug (12) und einem Sumpfabzug (5) betrieben. Das Rücklaufverhältnis, bezogen auf den Seitenabzug, betrug etwa 1,7. Aus dem Sumpfabzug (5) wurde Wasser abgeleitet.

Der Kolonne (1) wurde als Seitenabzug (6) ein wasserhaltiges THF mit einem Wassergehalt von 5 bis 6 % entnommen, das noch 40% des ursprünglichen Gehaltes an Dihydrofuran und alle Methyltetrahydrofurane enthielt.

Als Kopfabzug (12) wurde etwas über 0,1% des Zulaufs entnommen. Der Kopfabzug hatte eine THF-Konzentration von 20 %, eine Konzentration an Dihydrofuran von etwa 70% und enthielt über 80 bis 90 % des zugeströmten Dihydrofurans bzw. der Leichtsiederverunreinigungen.

Der Seitenabzug (6) der ersten Kolonne wurde unter Druckerhöhung der zweiten Kolonne (2) zugeführt, die bei 5 bar und mit einem Rücklaufverhältnis von ca. 1 betrieben wurde. Der Kopfabzug (7) der zweiten Kolonne, der praktisch alles Wasser und restliche Leichtsieder enthielt, wurde zur ersten Kolonne (1) zurückgeführt. Der Sumpfabzug, der sich als praktisch wasserfrei erwies und noch etwa 40 ppm Dihydrofuran enthielt, wurde in die drittel Kolonne (3) weitergeleitet.

Die dritte Kolonne, die sog. Reinkolonne, wurde mit einem Rücklaufverhältnis von etwa 5, bezogen auf den Seitenabzug, betrieben. Das dem Seitenabzug (11) entnommene Reinst-THF hatte eine Reinheit von 99,99% und enthielt weniger als 30 ppm Dihydrofuran und 10 ppm Methyltetrahydrofuran.

Als Kopfabzug wurden der dritten Kolonne (3) 1 bis 2% des THF-Zulaufs entnommen und die die erste Kolonne (1) zurückgeführt. Er enthielt 200 ppm Dihydrofuran. Als Sumpfabzug (9) wurden 0,6 bis 0,7% des Zulaufs (8) entnommen. Der Sumpfabzug enthielt praktisch alles Methyltetrahydrofuran in 80%-iger Konzentration.

## Patentansprüche

1. Verfahren zur destillativen Reinigung von rohem wasserhaltigen Tetrahydrofuran, bei dem man das rohe Tetrahydrofuran durch drei Destillationskolonnen leitet, wobei man Wasser aus dem Sumpf der ersten Kolonne abzieht, wasserhaltiges Tetrahydrofuran vom Kopf der zweiten Kolonne in die erste Kolonne zurückführt und aus dem Sumpfprodukt der zweiten Kolonne in der dritten Kolonne reines Tetrahydrofuran abdestilliert, dadurch gekennzeichnet, daß man einen Seitenabzug der ersten Kolonne in die zweite Kolonne leitet, das Kopfprodukt der dritten Kolonne in die erste Kolonne zurückführt, am Kopf der ersten Kolonne ein Destillat entnimmt, und das reine Tetrahydrofuran aus dem Seitenabzug der dritten Kolonne gewinnt.

2. Verfahren nach Anspruch 1, dadurch gekennzeichnet, daß man am Kopf der ersten Kolonne ein Destillat entnimmt, das nicht mehr als 50 Gew.% Tetrahydrofuran enthält.

## Claims

1. A process for the purification of crude water-containing tetrahydrofuran by distillation, in which the crude tetrahydrofuran is passed through three distillation columns, water being removed from the bottom of the first column, water-containing tetrahydrofuran being recycled from the top of the second column into the first column and pure tetrahydrofuran being distilled off from the bottom product of the second column, in the third column, wherein a side stream of the first column is passed into the second column, the top product of the third column is recycled to the first column, a distillate is removed at the top of the first column, and the pure tetrahydrofuran is obtained from the side take-off of the third column.

2. A process as claimed in claim 1, wherein a distillate which contains not more than 50% by weight of tetrahydrofuran is removed at the top of the first column.

## Revendications

1. Procédé de purification par distillation de tétrahydrofuranne contenant de l'eau brut, dans lequel on fait passer le tétrahydrofuranne brut dans trois colonnes de distillation, on prélève de l'eau en bas de la première colonne, on recycle le tétrahydrofuranne contenant de l'eau de la tête de la deuxième colonne dans la première colonne et on distille du tétrahydrofuranne pur dans la troisième colonne à partir du produit du bas de la deuxième colonne, caractérisé en ce qu'on fait passer dans la deuxième colonne un produit de prélèvement latéral de la première colonne, on recycle dans la première colonne le produit de tête de la troisième colonne, on prélève un distillat en tête de la première colonne et on obtient le tétrahydrofuranne pur par un prélèvement latéral dans la troisième colonne.

2. Procédé selon la revendication 1, caractérisé en ce qu'on prélève en tête de la première colonne un distillat qui ne contient pas plus de 50% en poids de tétrahydrofuranne.

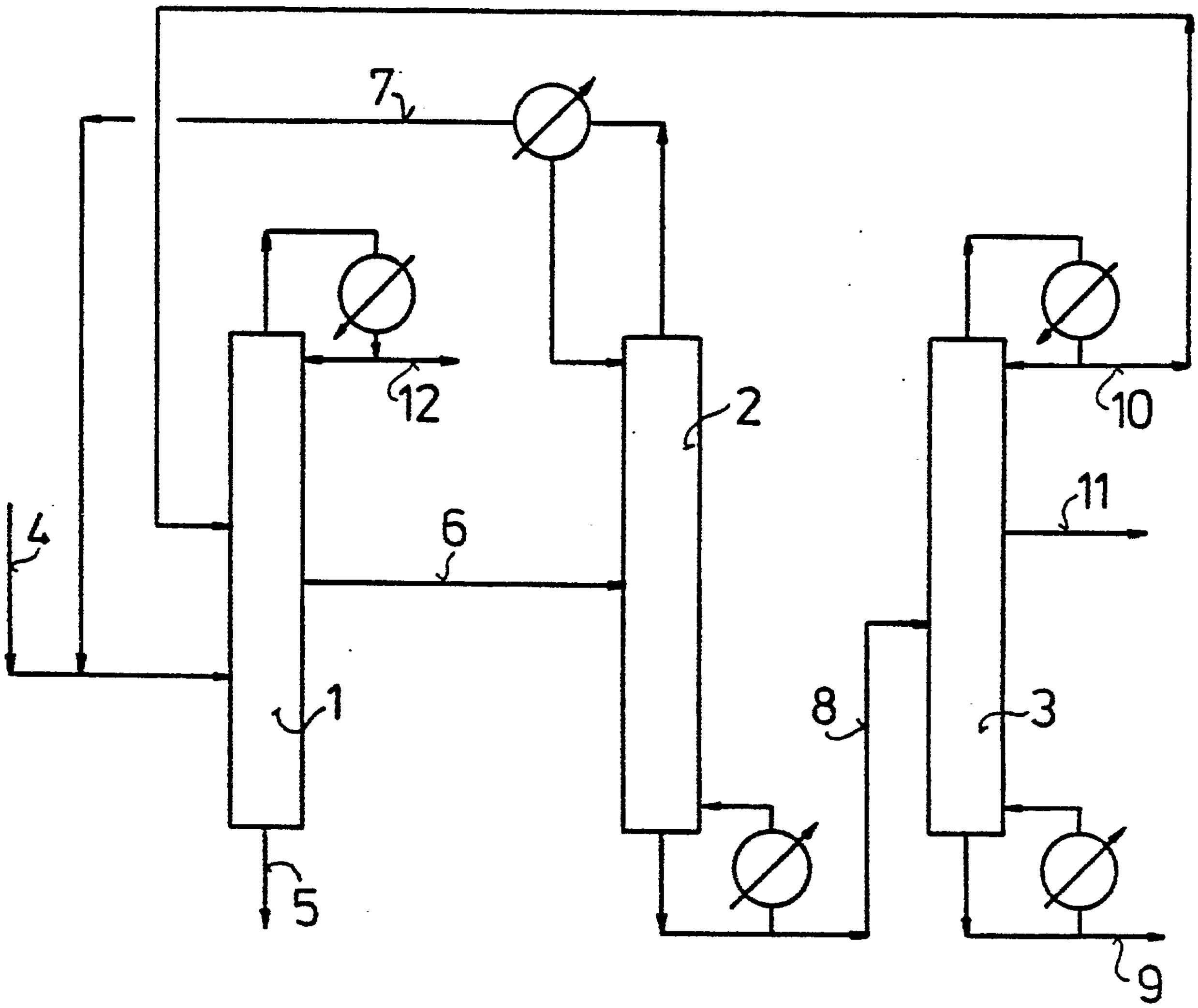
7
12
2
10
11
4
6
1
8
3
5
9